# EUROPEAN PATENT APPLICATION

(11) **EP 3 741 222 A1**
(43) Date of publication of application: **25.11.2020**
(21) Application number: 20460006.8
(22) Date of filing: 29.01.2020
(51) Int. Cl.: A23L 33/135, A23B 4/00, C12N 1/20

(54) **METHOD OF ACTIVATING MIXTURE OF LYOPHILIZED ACIDOPHILIC BACTERIA OF MILK ACID INTENDED FOR LOW TEMPERATURE DECONTAMINATION OF OCHRATOXIN A IN FOOD BLOOD**

(30) Priority: 22.05.2019 PL 43000119
(71) Applicant: University of Science and Technology, 85-796 Bydgoszcz (PL)
(72) Inventor: Grzelakowska, Agnieszka, 86-031 Osielsko (PL); Grajewski, Jan, 85-667 Bydgoszcz (PL); Twaruzek, Magdalena, 85-782 Bydgoszcz (PL); Banach-Szott, Magdalena, 86-086 Lisi Ogon (PL)

(57) **Abstract**

The idea of the invention is a method of activating mixture of lyophilized acidophylic bacteria of milk acid - *Lactobacillus acidopfililus* intended for low temperature decontamination of ochratoxin A mycotoxin in food pork blood in connection with the presence of mould (storage) fungi in the raw material of *Aspergillus and*/*or Penicillium* type. Application of a prebiotic from the group of oligofructans with molecular mass of 5000 Da to activate the mixture of lyophilized acidophylic bacteria of milk acid increases water absorption and gelation ability as well as favourably influences its consistency and structure of the food blood. The prebiotic provides cream like consistency and velvet feel of the product. It influences favourably the consistency of frozen products preventing release of water from a product after its defreezing. During the implementation of the invention, a considerable activation of milk acid bacteria intended for low temperature decontamination of ochratoxin A appearing in food pork blood was unexpectedly observed after the application of an optimal amount of 20 ug.1cm-3 mixture of a chemical compound from the group of purines, with molecular formula of C5H5N5 and molar mass of 135.13 g/mol and molecular formula of C17H20N4O6, molar mass of 376.36 g/mol, in the optimum amount of 1,5 µg.1cm-3.

## Description

The idea of the invention is the method of activating a mixture of lyophilized acidophylic bacteria of milk acid - *Lactobacillus acidopfililus* intended for low temperature decontamination of the mycotoxin - ochratoxin A contained in food pork blood in connection with its presence in raw material of mould (storage) fungi of *Aspergillus* and/or *Penicillium* type and destined for application in food industry to increase safety of food blood by decreasing the content of ochratoxin A in the raw material.

From the specialist literature we are aware of huge adaptability of heterotrophs to their surrounding environment and that they may be adapted to use various substrates in wide temperature range. It was found out that optimation of conditions at the growth stage of acidophilic cells of *Lactobacillus acidopfililus* milk acid bacteria has very significant impact on the efficiency of decontamination of ochratoxin A.

We know from the patent description no PL 380394 an agent for mycotoxin biodegradation such as deoxynivalenol (DON), nivalenol (NIV), toxin T-2 and toxin HT-2 as well as other toxins such as zearalenone and fumonisin. The idea of the invention consisted in applying the agent during grain conditioning in a mill in a combined grain scrubber to separate contaminants; this operation allows for a contact between grain's surface with milk acid bacteria. We also know from patent description no PL 226236 a method of preparing vaccine of probiotic bacteria as a starter culture in meat products. However, it is characterized by excessive, as for the needs of food blood processing industry, temperature of both incubation of starter culture at 37°C as well as temperature of maturing process of meat processed products at 18°C.

In the method according to the invention no PL 225124 it has been declared that as a result of extending the time of interacting on the blood by standarized starter cultures, compound population of milk bacteria of *Lactobacillus acidophilus,* decreasing of temperature and applying various additions one may increase efficiency of ochratoxin degradation by 50% as compared to its exit value. The effect of decreasing the content of ochratoxin A in pig blood was obtained in the invention by unexpected decreasing of blood temperature below 10°C, slowing down of fermentation and extending the time of interaction by milk acid bacteria with blood components till 96 hours.

The specialist literature (SIONEK B., KOLOZYN-KRAJEWSKA D., PASTERNOK I. : ŻYWNOŚĆ. Nauka. Technologia. Jakość, 2014, 1 (92), 103 - 113) revealed very good survival potential of bacteria with probiotic features in raw maturing sausages during their cold room storing for 230 days in the temperature of 4-6°C. The number of milk bacteria (LAB) marked after 30, 60, 90, 150, 180 and 230 days of fermentation in examined sausages ranged between 108 and 109. Also in the case of yoghurts frozen to 6°C ±0,5°C and stored for 12 weeks, good survival potential of microflora was revealed, which made it possible to obtain products meeting the criteria of therapeutic minimum (probiotic population of over 6 log of cfu/g) over the whole period of conducting examination.

No method of activating a mixture of lyophilized acidophylic bacteria of milk acid intended for low temperature decontamination of ochratoxin A contained in food blood is known according to the elaborated invention.

The idea of the invention is the method of preparing *Lactobacillus acidopfililus* bacteria for low temperature, prolonged fermentation as a way to increase safety of food blood by decreasing the content of ochratoxin A in the raw material.

The idea of activating a mixture of lyophilized acidophilic bacteria of milk acid intended for low temperature decontamination of ochratoxin A in food blood consists in the fact that to the mixture of lyophilized acidophilic bacteria of milk acid, a prebiotic from the group of oligofructans is added that is made up of 30-35 simple sugars connected by β-2,1-glycosidic bonds into a linear chain with molecular mass of 5000 Da, in the amount of 0.8-1.5 g for 1 dm-3 of the mixture, with organic growth factors of the milk acid bacteria having the form of the mixture of chemical compounds from the group of purines with molecular formula of C5H5N5 and molar mass of 135.13 g/mol, within the range of 15 to 25 µg.1cm-3 of the mixture and molecular formula of C17H20N4O6, molar mass of 376.36 g/mol, 0,1 do 1,5 µg.1cm-3, whereas the mixture is incubated within 24-48 hours in the temperature of 4-10°C.

The method of activating lyophilized starter cultures according to the invention consists in the fact that in known bioreactors, lyophilisate (Lyofast Protective - 1 dose (1011 cfu) containing 1*106 cfu/portion of *Lactobacillus acidopfililus, Lactobacillus rhamnosus* and *Lactobacillus plantarum* bacteria was diluted in 100 cm3 of fresh pork blood, haemolysed with dihydrate hydrate of disodium citrate 20% solution of sodium citrate: 5 cm3/100 cm3 of blood stabilized with sodium chloride in the concentration of NaCl in blood of 1%, in the presence of 1.0 g of prebiotic - polysaccharide belonging to oligofructans, made up of of 30-35 simple sugars connected by β-2,1-glycosidic bonds into a linear chain. The molecular mass of the polysaccharide amounts to 5000 Da. In one's own reserach, considerable activation of milk acid bacteria was found out unexpectedly after application of 20 ug.1cm-3 of the solution of the mixture of the chemical compound from the group of purines with molecular formula of C5H5N5 and molar mass of 135.13 g/mol and the compound of molecular formula of C17H20N4O6 and molar mass of 376,36 g/mol in the amount of 0,1 µg.1cm-3 of the solution. The mixture was incubated for the period from 24 to 48 hours, in the known bioreactors, preferably for 24 hours with temperature and CO2 adjustment. The bacteria that started to reproduce after the time span called the delay period were adapted to new conditions - temperature, amount of nutrition components. Fermentation being the way of decontaminating of ochratoxin A in low temperature has not been stopped. After the end of the incubation time, 100% growth of cfu/portion of the milk acid bacteria was detected.

The advantage of activating mixture of lyophilized acidophilic bacteria of milk acid intended for low temperature decontamination of ochratoxin A in food blood is the possibility of applying active process of dissolution of ochratoxin A in food blood within the range of low temperatures (4.0 to 10.0 °C), which was not possible within so far known, optimal temperature ranges (28,0 - 36,0 °C). Due to joint interaction of all factors, and especially the application of special organic growth factors of bacteria, efficiency of ochratoxin A biodegradation in the raw material was established at 60-70% as compared to the content of the original value. Survival potential of bacteria with probiotic features in food blood resembles product maturing process during cold room storage. During the incubation, milk acid bacteria (*Lactobacillus rhamnosus* and *Lactobacillus plantarum*) take up the process of fermentation, which results in partial dissolution of ochratoxin A. This procedure increases toxicological safety of produced product. Using of a prebiotic from the group of oligofructans increases the ability to absorb water and gelation and also influences favourably consistency and structure of food blood. As a substance substituting fat in production, it provides adequate cream consistency and velvet texture. Addition of this oligofructan also influences favourably the consistency of frozen products.

The method according to the invention was presented in detail in the following examples.

### Example 1.

In the known bioreactors, lyophilisate (Lyofast Protective - 1 dose (1011 cfu) containing 1*106 cfu/portion of *Lactobacillus acidopfililus, Lactobacillus rhamnosus* and *Lactobacillus plantarum* bacteria was diluted in (calculation for) 100 cm3 of fresh pork blood, haemolysed with dihydrate hydrate of disodium citrate 20% solution of sodium citrate: 5 cm3/100 cm3 of blood stabilized with sodium chloride in the concentration of NaCl in blood of 1%, in the presence of 1.0 g of oligofructan with molecular mass of 5000 Da. Then, to the solution prepared above, a mixture containing 20 µg.1cm-3 of the chemical compound from the group of purines, with molecular formula of C5H5N5 and molar mass of 135.13 g/mol and 0.1 µg.1cm-3 of the compound with molecular formula of C17H20N406 and molecular mass of 376.36 g/mol being organic growth factors were added. The mixture was incubated for 24 hours in the known bioreactors with temperature and CO2 adjustment in the temperature of 10°C.

### Example 2.

In the known bioreactors, lyophilisate (Lyofast Protective - 1 dose (1011 cfu) containing 1*106 cfu/portion of *Lactobacillus acidopfililus, Lactobacillus rhamnosus* and *Lactobacillus plantarum* bacteria was diluted in (calculation for) 100 cm3 of fresh pork blood, haemolysed with dihydrate hydrate of disodium citrate (20% solution of sodium citrate: 5 cm3/100 cm3 of blood) stabilized with sodium chloride (concentration of NaCl in blood of 1%), in the presence of 1.5 g of oligofructan with molecular mass of 5000 Da. Then, to the solution prepared above, a mixture containing of 25 µg.1cm-3 of the chemical compound from the group of purines, with molecular formula of C5H5N5 and molar mass of 135.13 g/mol and 0.5 µg.1cm-3 of the compound with molecular formula of C17H20N4O6 and molecular mass of 376.36 g/mol being organic growth factors were added. The mixture was incubated for 36 hours in the known bioreactors with temperature and CO2 adjustment in the temperature of 6°C.

### Example 3.

In the known bioreactors, lyophilisate (Lyofast Protective - 1 dose (1011 cfu) containing 1*106 cfu/portion of *Lactobacillus acidopfililus, Lactobacillus rhamnosus* and *Lactobacillus plantarum* bacteria was diluted in (calculation for) 100 cm3 of fresh pork blood, haemolysed with dihydrate hydrate of disodium citrate (20% solution of sodium citrate: 5 cm3/100 cm3 of blood) stabilized with sodium chloride (concentration of NaCl in blood of 1%), in the presence of 0.8 g of oligofructan with molecular mass of 5000 Da. Then, to the solution prepared above, a mixture containing 15 µg.1cm-3 of the chemical compound from the group of purines, with molecular formula of C5H5N5 and molar mass of 135.13 g/mol and 1.5 µg.1cm-3 of the compound with molecular formula of C17H20N4O6 and molecular mass of 376.36 g/mol being organic growth factors were added. The mixture was incubated for 48 hours in the known bioreactors with temperature and CO2 adjustment in the temperature of 4°C.

## Claims

1. Method of activating mixture of lyophilized acidophylic bacteria of milk acid intended for low temperature decontamination of ochratoxin A in food blood in the known bioreactors that is **characterized by** the fact that to the mixture of lyophilized acidophylic bacteria of milk acid a prebiotic is added from the group of oligofructans, made up of 30-35 simple sugars connected by β-2,1-glycosidic bonds into a linear chain with molecular mass of 5000 Da, in the amount of 0.8-1.5 g for 1 dm-3 of the mixture.

2. The method described in item 1 is **characterized by** the fact that organic growth factors of milk acid bacteria were applied in the form of mixture of chemical compounds from the group of purines with molecular formula of C5H5N5 and molar mass of 135.13 g/mol, within the range of 15 to 25 µg.1cm-3 of the mixture and molecular formula of C17H20N4O6, molar mass of 376.36 g/mol, 0,1 do 1,5 µg.1cm-3.

3. The method described in item 1 is **characterized by** the fact that the mixture is incubated for 24-48 hours in the temperature of 4-10°C.
